# EUROPEAN PATENT SPECIFICATION

(11) **EP 4 026 576 B1**
(45) Date of publication and mention of the grant of the patent: **04.12.2024**
(21) Application number: 20871078.0
(22) Date of filing: 01.10.2020
(51) Int. Cl.: A61M 16/00, A61M 16/16

(54) **HUMIDIFIER AND RESPIRATORY ASSIST DEVICE**
BEFEUCHTER UND BEATMUNGSHILFSVORRICHTUNG
HUMIDIFICATEUR ET DISPOSITIF D'ASSISTANCE RESPIRATOIRE

(30) Priority: 02.10.2019 JP 2019182402; 02.10.2019 JP 2019182403
(43) Date of publication of application: 13.07.2022
(60) Divisional application: 24203503.8
(73) Proprietor: Magos Co., Ltd., Kawaguchi-shi, Saitama 332-0015 (JP)
(72) Inventor: NITTA Kazufuku, Kawaguchi-shi, Saitama 332-0015 (JP)
(74) Representative: TBK
(86) International application number: PCT/JP2020/037474
(87) International publication number: WO 2021/066114

(56) References cited:
- WO-A1-2020/045096
- AU-A1- 2015 204 372
- CN-U- 204 446 851
- CN-U- 206 548 963
- JP-A- 2016 168 341
- JP-A- 2018 514 301
- JP-A- H08 121 701

## Description

### Technical Field

The present invention relates to a humidification device that humidifies a feed gas which is to be fed to a user, and a respiratory assistance device including the humidification device.

### Background Art

Automatic ventilation devices designed to regulate or assist ventilation by connecting to users' respiratory tracts are widely used in medical practice, which include respiratory assistance devices to be used for continuous positive airway pressure therapy (nasal CPAP), a treatment for sleep apnea syndrome.

Continuously feeding a dry gas to a user's respiratory tract can cause discomfort to the user, and in some cases can even trigger damage to the respiratory tract. Therefore, the respiratory assistance device is connected to a humidification device that adds moisture to a feed gas.

Conventional humidification devices used in respiratory assistance devices usually adopt a method in which a heating element (heater plate) heats an entire body of water in a reservoir and vaporizes the water (see, for example, PCT Patent Application Publication No. WO/2007/019625, Japanese Translation No. JP 2009-504277 A).

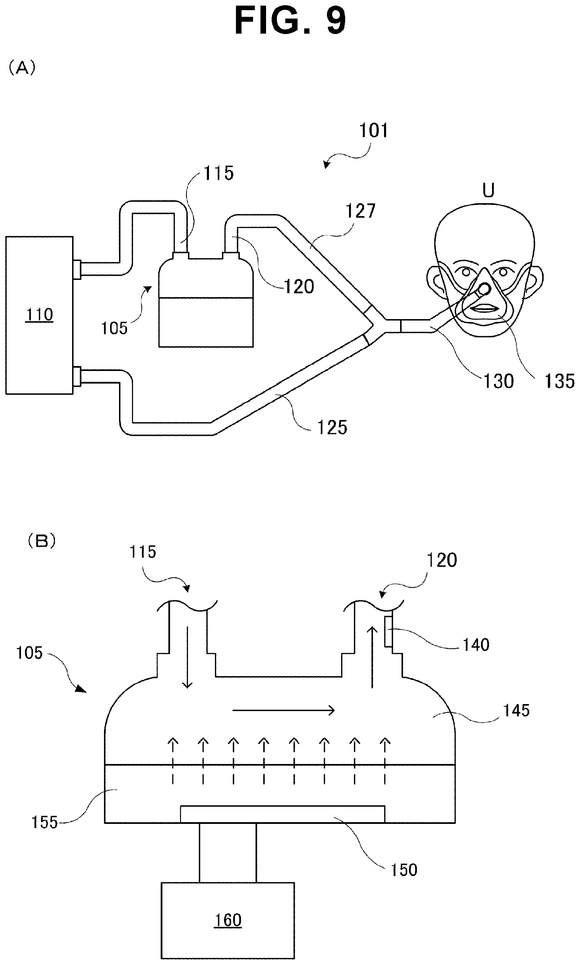

FIG. 9A illustrates a schematic diagram of a conventional respiratory assistance device 101. The respiratory assistance device 101 includes a humidification device 105 that utilizes a heating and vaporization method. The humidification device 105 takes in a feed gas fed from a ventilator 110 through a feed gas inlet 115, heats and humidifies the feed gas, and sends out the feed gas through a feed gas outlet 120 to an inspiratory side respiratory circuit 127. The feed gas is fed through a hose 130 from an interface 135 to a user U. Expiratory air is released to the outside through an expiratory side respiratory circuit 125.

FIG. 9B is an explanatory view of the conventional humidification device 105. The humidification device 105 stores liquid (water) 155 inside a case. The liquid (water) 155 is heated and vaporized by a heating element 150. The heating element 150 has, for example, an electric resistance element (not illustrated), and is heated by the application of electric current from a power supply 160.

More specifically, the feed gas flowing from the feed gas inlet 115 contains water vapor which has been vaporized from a surface of the liquid (water) 155 within a humidification space 145. After being humidified, the feed gas is sent from the feed gas outlet 120 through the respiratory circuit to the respiratory tract of the user U. At this time, the outlet temperature of the feed gas is measured by a feed gas outlet temperature measurement unit 140, and power which is to be inputted to the heating element 150 is controlled so that appropriate temperature and humidity are achieved in the respiratory tract of the user U.

Furthermore, AU 2015 204 372 A1 discloses a humidifier comprising a controller which is configured to determine an ambient humidity, calculate an amount of liquid to be added to the flow of breathable gas based on the determined ambient humidity and the target humidity, calculate an amount of energy required to vaporize the amount of liquid, and control the water supply unit to deliver the amount of liquid to the porous heating element.

### Summary of Invention

### Technical Problem

However, in the technology described in JP 2009-504277 A, the temperature of the water in the entire reservoir must be increased to generate a sufficient amount of water vapor. Therefore, energy consumption is high and the time necessary until humidification becomes possible is lengthened. It is also difficult to reduce the size of the device because a large amount of hot water has to be stored. In addition, since a large amount of hot water needs to be stored, there is a great risk of the hot water leaking when the humidification device tips over, resulting in scald to the user or the like. In the future, the respiratory assistance devices are expected to be used more often in home medical care, and in such cases, it is inconvenient for family members other than medical personnel to handle such devices.

The present invention was made in consideration of the above-described problems, and an object thereof is to provide a humidification device that can be made smaller in size and lighter in weight, and that can quickly and sufficiently humidify a gas without heating an entire body of water to be stored, as well as a respiratory assistance device.

### Solution to Problem

According to the invention this object is achieved by humidification device according to claim 1 and a humidification method according to claim 10. Further features and advantageous modifications are shown in the dependent claims.

According to the present invention, it is possible to achieve extremely excellent effects of enabling high-speed heating and humidification control and humidification control with minimum liquid supply, by independently performing controlling the input power by referring to the temperature in the respiratory circuit connected to the humidification device, calculating the target input power, as a target, corresponding to the target heated and humidified state of the feed gas, and controlling the supply amount of the liquid on the basis of the difference value between the input power and the target input power.

According to the invention, since the liquid (water) can be supplied to the heating unit only in an amount required for heating and humidification, it is possible to eliminate the accumulation of the liquid water and prevent bacterial growth, which achieves the excellent effect of providing a humidification device which is also excellent

According to the invention, it is possible to achieve the excellent effect of enabling to provide a respiratory assistance device including the compact and lightweight humidification device that can perform quick heating and humidification with a minimum amount of liquid (water amount) and an optimum input power.

(16) The present invention also provides a respiratory assistance method including the humidification method described in the claims.

In this regard, it is possible to achieve extremely excellent effects of enabling high-speed heating and humidification control and humidification control with a minimum liquid supply, by independently performing controlling the input power by referring to the temperature in the respiratory circuit connected to the humidification device, calculating the target input power, as a target, corresponding to the target heated and humidified state of the feed gas, and controlling the liquid supply unit on the basis of the difference value between the input power and the target input power. Advantageous Effects of Invention

The humidification device, the respiratory assistance device, and the humidification method according to claims 1 to 16 of the present invention have beneficial effects of realizing a humidification device that is easy to perform maintenance, hygienic, compact in size, light in weight, and capable of quick heating and humidification, and a respiratory assistance device including such a humidification device. Brief Description of Drawings

FIG. 1 is a schematic diagram of a respiratory assistance device according to an embodiment of the present invention.
FIG. 2A is an explanatory view of an inside of a case of a humidification device in the respiratory assistance device according to the embodiment of the present invention. FIG. 2B is a cross-sectional view of the humidification device.
FIG. 3A is an explanatory view of the humidification device in the respiratory assistance device according to the embodiment of the present invention. FIG. 3B is an explanatory view of a humidification device of a respiratory assistance device according to a first variation of the present invention.

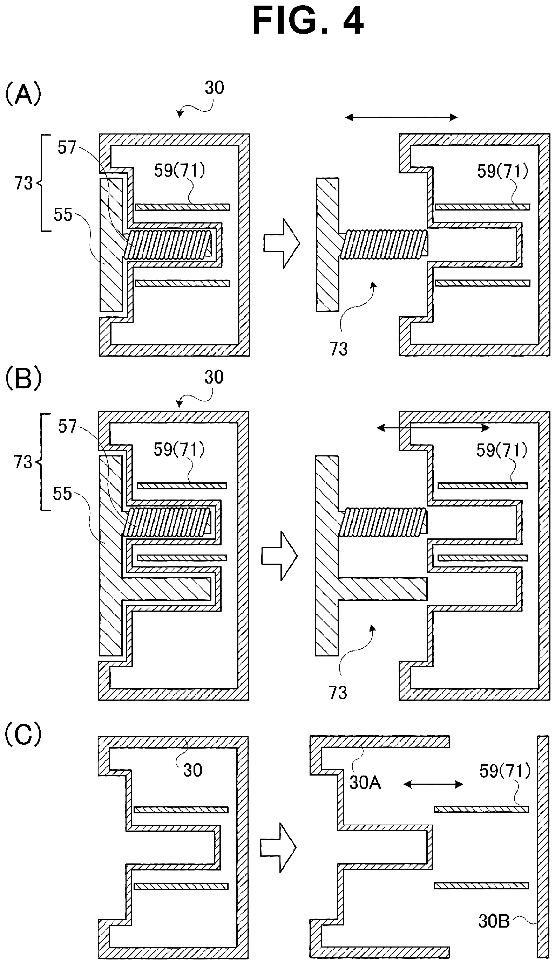

FIG. 4A is an explanatory view illustrating an operation of separating an energy supply unit and a humidification unit that constitute the humidification device in the respiratory assistance device according to the embodiment of the present invention. FIG. 4B is an explanatory view illustrating an operation of separating an energy supply unit and a humidification unit that constitute the humidification device in the respiratory assistance device according to the first variation of the present invention. FIG. 4C is an explanatory view illustrating an aspect of opening a humidification space within a case and separating only the humidification unit for maintenance.
FIG. 5A is an explanatory view of an inside of a case of a humidification device in a respiratory assistance device according to a second variation of the present invention. FIG. 5B is a cross-sectional view of the case of the humidification device.
FIG. 6 is a flowchart that explains an algorithm for controlling an outlet temperature of a feed gas in the humidification device in the respiratory assistance device.
FIG. 7 is a flowchart that explains an algorithm for calculating and setting a target input power in real time in the humidification device in the respiratory assistance device.
FIG. 8 is a flowchart that explains an algorithm for controlling the amount of liquid (water) to be supplied from a power supply to a heating unit.
FIG. 9A is a schematic diagram of a conventional respiratory assistance device. FIG. 9B is an explanatory view of a conventional humidification device.

### Description of Embodiments

An embodiment of the present invention will be described below with reference to the accompanying drawings.

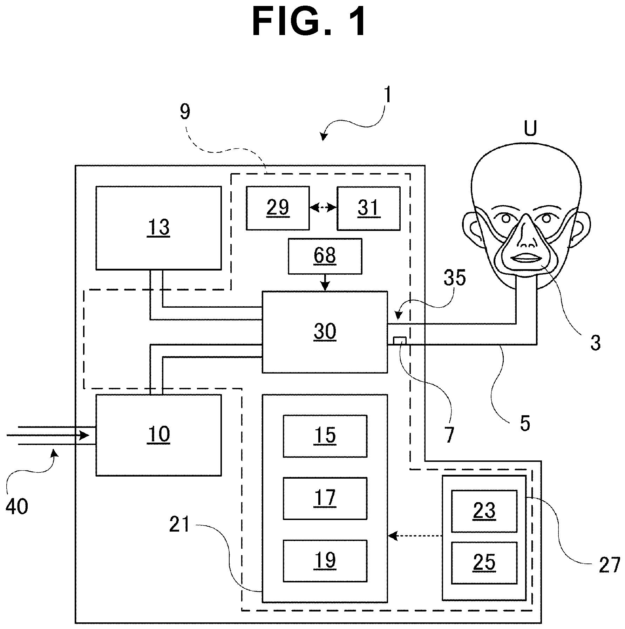

FIGS. 1 to 8 illustrate an example of the embodiment of the present invention, and the identical reference numerals indicate the same objects in the drawings. In each figure, part of the configuration is omitted, as appropriate, to simplify the drawings. The size, shape, thickness, and the like of the members are exaggerated as appropriate.

FIG. 1 is a schematic diagram of a respiratory assistance device 1 according to the embodiment of the present invention. The respiratory assistance device 1, which assists respiration of a user U, includes a blower 10 configured to send out a feed gas, a medical gas cylinder 13 configured to supply a medical gas to be used for medical treatment or the like, a humidification device 9 configured to humidify the feed gas, a respiratory circuit 5 configured to lead the feed gas to the user U, and an interface 3 for respiratory assistance, placed near the nose and/or oral cavity of the user U, that is configured to lead the feed gas.

The blower 10 draws air from an inlet 40 and feeds the air into a humidification device 30. The medical gas supplied from the medical gas cylinder 13 is also fed into the humidification device 30. In other words, the feed gas may be a mixed gas of the air drawn from the inlet 40 and the medical gas.

The medical gas may be an oxygen gas, for example.

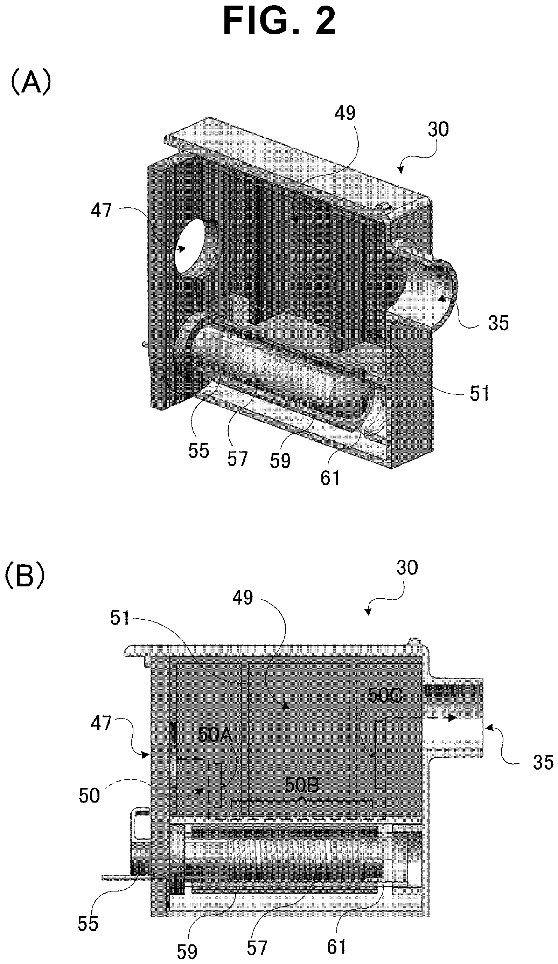

The humidification device 9 has a case 30, a power supply 29, an input power measurement unit 31 configured to measure an input power to be inputted from the power supply 29 to a heating unit (metal porous body) 59 (see FIG. 2A, which will be described later), a liquid supply device 68 configured to supply a liquid (water) to the heating unit (metal porous body) 59, an outside environmental variable measurement unit 27 configured to measure environmental variables of an environment where the user is present, and a control device 21 configured to control the humidification device 9. At a feed gas outlet 35 to which the feed gas is sent out from the case 30 (see FIG. 2A, which will be described later), a feed gas outlet temperature measurement unit 7 configured to measure the temperature of the feed gas to be sent to the respiratory circuit 5 is disposed.

The location of this "outlet" is not specifically limited, and may be located anywhere downstream from the heating unit (metal porous body) 59.

The power which is to be inputted from the power supply 29 is to be directly sent to a coil 57, but the electric energy is inputted to the heating unit (metal porous body) 59 (see FIG. 2A, which will be described later) through an electromagnetic induction phenomenon, and so the above-mentioned expression is adopted. Therefore, the input power measurement unit 31 may measure either the power which is to be inputted to the coil 57 or the energy which is to be transferred to the heating unit (metal porous body) 59.

The heating unit 59 is a metal porous body with a porous structure containing metal. The heating unit (metal porous body) 59 may have a mesh structure made of pressed metal fibers.

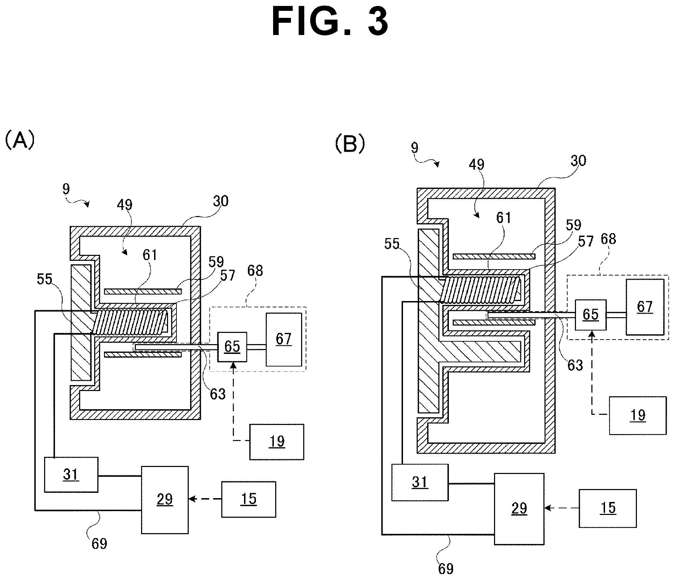

The control device 21 includes a heating control unit 15 configured to control the input power by referring to a temperature within the humidification device 9 or within the respiratory circuit 5 connected to the humidification device 9, a target input power calculation unit 17 configured to calculate a target input power, as a target, corresponding to a target heated and humidified state of the feed gas, and a liquid supply control unit 19 configured to control the amount of liquid (see FIG. 3, which will be described later) on the basis of a difference value between the measured input power and the target input power.

The outside environmental variable measurement unit 27 has an outside temperature measurement unit 23 configured to measure the temperature of an environment in which the respiratory assistance device 1 is installed, and an outside humidity measurement unit 25 configured to measure the humidity of the environment in which the respiratory assistance device 1 is installed. The outside temperature measurement unit 23 may be, for example, a resistance thermometer. The outside humidity measurement unit 25 may be, for example, a bimetal type, or a digital type hygrometer which uses a moisture sensing agent and comb electrodes.

The control device 21 is constituted of a CPU, a RAM, a ROM, and the like, and performs various types of control. The CPU is a so-called central processing unit, and executes various programs to realize various functions. The RAM is used as a work area and a storage area for the CPU, and the ROM stores an operating system and the programs to be executed by the CPU.

The control device 21 may control the operation of the entire respiratory assistance device 1.

FIG. 2A is an explanatory view of the inside of the case 30 of the humidification device 9 in the respiratory assistance device 1 according to the embodiment of the present invention. The case 30 has a feed gas inlet 47 into which the feed gas is fed, the feed gas outlet 35 through which the feed gas is sent out to the respiratory circuit 5 (see FIG. 1), the coil 57 configured to generate a magnetic field, the heating unit (metal porous body) 59, an insulating unit 61 configured to provide electrical insulation between the coil 57 and the heating unit (metal porous body) 59, a ferrite 55 which is a magnetic body configured to increase efficiency of magnetic coupling between the coil 57 and the heating unit (metal porous body) 59, and a rectifying plate 51 configured to change a flow of the feed gas so that the feed gas is brought into contact with the heating unit (metal porous body) 59 for a sufficient length of time. The feed gas is heated and humidified by a flow of heated vapor which is generated in the heating unit (metal porous body) 59 and transferred into a humidification space 49.

FIG. 2B is a cross-sectional view of the humidification device 9. The feed gas is taken into the case 30 from the feed gas inlet 47, and the flow of the feed gas is altered by the rectifying plate 51. Then, the feed gas travels along, for example, a humidifying and heating path 50 as illustrated by a dashed line in FIG. 2B, which specifically includes an entry side path 50A from the feed gas inlet 47 to the heating unit (metal porous body) 59, a heating body side path 50B in the vicinity of the heating unit (metal porous body) 59, and an outlet side path 50C from the heating unit (metal porous body) 59 to the feed gas outlet 35. The feed gas is heated and humidified in the humidification space 49, by introduction of the heated vapor generated in the heating unit (metal porous body) 59, and is thereafter sent through the feed gas outlet 35 to the respiratory circuit 5.

The humidification device 9 includes the case 30 which contains the humidification space 49 in which the water vapor is introduced to the feed gas to be fed to the user U. The heating unit 59, the coil 57, and the insulating unit 61 are disposed in the humidification space 49. In addition, there is a liquid supply unit 63 (see FIG. 3, which will be described later) that supplies a liquid, to be vaporized and made into the water vapor, to the heating unit 59.

Specifically, the heating unit (metal porous body) 59 has a cylindrical shape with a perfect circular cross section. The heating unit (metal porous body) 59 contains metal and has electrical conductivity as a whole, while having sufficient electrical resistance so as to generate heat when electric current induced by electromagnetic induction from the coil 57 flows therethrough. The coil 57 is made of a metal wire wound in a spiral shape and is disposed along the outer periphery of the ferrite 55. The coil 57 has high electrical conductivity. The insulating unit 61 is disposed between the heating unit (metal porous body) 59 and the coil 57 to electrically insulate the heating unit (metal porous body) 59 and the coil 57 from each other. The insulating unit 61 also serves as an isolation wall that spatially isolates the coil 57 from the liquid (water) supplied from the liquid supply device 68 to the heating unit (metal porous body) 59 and the vapor from the liquid. The insulating unit 61 may be made of glass or a synthetic resin.

The coil 57 is disposed in the inner periphery of the heating unit 59 via the insulating unit 61.

The insulating unit 61 has a cylindrical shape, and is disposed on an inner peripheral side of the heating unit 59, where the coil 57 is disposed on an inner peripheral side of the insulating unit 61.

The heating unit 59 may be disposed such that a central axis of the cylindrical shape of the heating unit 59 is oriented in a non-vertical direction. An aspect in which the central axis is horizontal is described here as a basic posture, but the advantage of this humidification device is that humidification and heating can be performed even when the posture is changed.

FIG. 3A is an explanatory view of the humidification device 9 in the respiratory assistance device 1 according to the embodiment of the present invention.

In this variation, the ferrite 55 is provided on the inner peripheral side of the coil 57 to increase the magnetic coupling between the heating unit (metal porous body) 59 and the coil 57. Part of the case 30 may perform the function of the insulating unit 61 (see FIG. 2A, as described above). The case 30 may be made of a synthetic resin, such as an ABS resin, for example.

The input power to the coil 57 is applied from the power supply 29 through a power supply line 69. The power supply 29 is controlled by the heating control unit 15. The value of the power being applied is measured in real time by the input power measurement unit.

The liquid (water) is supplied from the liquid supply device 68 to the heating unit (metal porous body) 59. Specifically, the liquid (water) is supplied from a liquid storage unit 67 through the liquid supply unit 63 to the heating unit (metal porous body) 59. The liquid (water) is supplied gradually from an end of the liquid supply unit 63 to an inner peripheral surface of the heating unit (metal porous body) 59. The liquid supply unit 63 is tubular, and it is desirable that the end of the liquid supply unit 63 is disposed along the inner peripheral surface of the heating unit (metal porous body) 59. A supply amount of the liquid (water) is regulated by a liquid supply amount regulation unit 65, which may be, for example, a piezoelectric pump. The liquid supply amount regulation unit 65 is controlled by the liquid supply control unit 19 (see FIG. 1).

The liquid supply unit 19 supplies the liquid so as to maintain a state in which the liquid does not accumulate in the vicinity of the heating unit 59. That is, the liquid is supplied to the extent not exceeding a maximum amount of the liquid that can be vaporized by the heating unit (metal porous body) 59.

FIG. 4A is an explanatory view illustrating an operation of separating an energy supply unit 73 and a humidification unit 71 that constitute the humidification device 9 in the respiratory assistance device 1 according to the embodiment of the present invention. While in a state of heating and humidifying, the energy supply unit 73 including the ferrite 55 and the coil 57 is disposed in a recess of the case 30, which contains the heating unit (metal porous body) 59 therein, and the coil 57 and the heating unit (metal porous body) 59 together form a magnetic circuit to enable heating of the heating unit (metal porous body) 59 by an electromagnetic induction phenomenon (see the left drawing of FIG. 4A). In the case of performing maintenance, specifically in the case of replacing the humidification unit 71 having the heating unit (metal porous body) 59 with a new one, the energy supply unit 73 and the humidification unit 71 can be spatially separated (see the right drawing of FIG. 4A).

FIG. 4C is an explanatory view illustrating an operation of opening the humidification space 49 in the case 30 and separating only the humidification unit 71 (heating unit (metal porous body) 59). The humidification device 30 has a main unit 30A and a lid unit 30B. During maintenance, specifically in the case of replacing the heating unit (metal porous body) 59 with a new one, the lid unit 30B is opened to easily remove and replace the heating unit (metal porous body) 59 with a new one (see the right drawing of FIG. 4C).

FIG. 3B is an explanatory view of a humidification device 9 of a respiratory assistance device 1 according to a first variation of the present invention.

In this variation, in order to close a magnetic circuit between a heating unit (metal porous body) 59 and a coil 57 as much as possible and increase magnetic coupling, a U-shaped ferrite 55 is provided. Part of a case 30 may perform the function of an insulating unit 61 (see FIG. 2A, as described above). The case 30 may be made of a synthetic resin, such as an ABS resin, for example.

An input power to the coil 57 is applied from a power supply 29 through a power supply line 69. The power supply 29 is controlled by a heating control unit 15. The value of the power being applied is measured in real time by an input power measurement unit.

A liquid (water) is supplied from a liquid storage unit 67 through a liquid supply unit 63 to the heating unit (metal porous body) 59. The supply amount of the liquid (water) is controlled by a liquid supply amount regulation unit 65, which may be, for example, a piezoelectric pump. The liquid supply amount regulation unit 65 is controlled by a liquid supply control unit 19 (see FIG. 1).

The liquid supply unit 19 supplies the liquid so as to maintain a state in which the liquid does not accumulate in the vicinity of the heating unit 59. The supply of the liquid to the heating unit (metal porous body) 59 is the same as in the case of FIG. 3A, and hence a description therefor will be omitted.

FIG. 4B is an explanatory view illustrating an operation of separating an energy supply unit 73 and a humidification unit 71 that constitute the humidification device 9 in the respiratory assistance device 1 according to the first variation of the present invention.
In a state of heating and humidifying, the energy supply unit 73 including the ferrite 55 and the coil 57 is disposed in a recess of the case 30, which contains the heating unit (metal porous body) 59 therein, and the coil 57 and the heating unit (metal porous body) 59 together form the magnetic circuit to enable heating of the heating unit (metal porous body) 59 by an electromagnetic induction phenomenon (see the left drawing of FIG. 4B). In the case of performing maintenance, specifically in the case of replacing the humidification unit 71 having the heating unit (metal porous body) 59 with a new one, the energy supply unit 73 and the humidification unit 71 can be spatially separated (see the right drawing of FIG. 4B).

Since the liquid (water) is supplied to the humidification unit 71, bacteria may occur and accordingly periodic replacement is desirable. Since the case 30 can be easily separated into the energy supply unit 73 and the humidification unit 71, replacement of just the humidification unit 71 with a new one is possible, which makes maintenance easy.

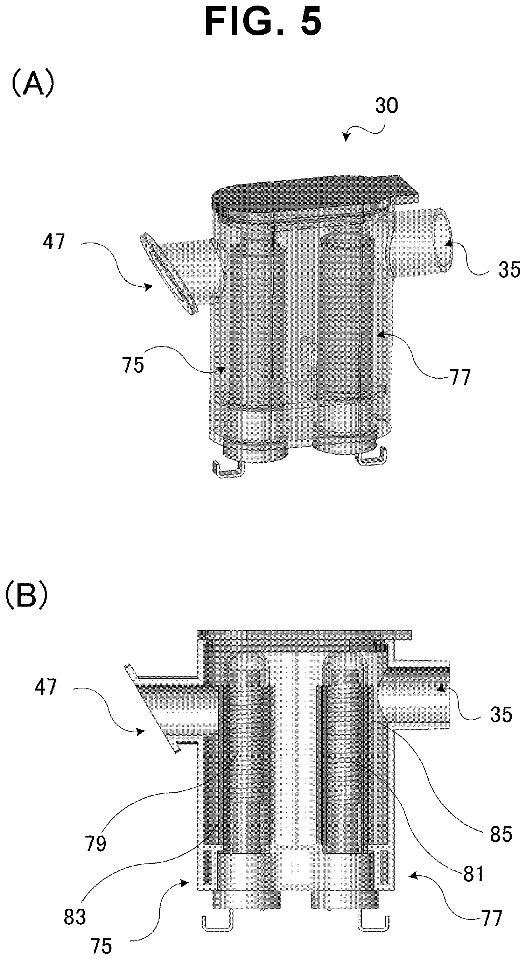

FIG. 5A is an explanatory view of an inside of a case 30 of a humidification device 9 in a respiratory assistance device 1 according to a second variation of the present invention. In this variation, a warming unit 75 which contains a heating unit (metal porous body) 59, and a humidification unit 77 which contains another heating unit (metal porous body) 59 similarly are provided. A feed gas introduced from a feed gas inlet 47 is heated by the warming unit 75 to which no liquid is supplied, and is humidified by the humidification unit 77 to which a liquid is supplied. After that, the feed gas is sent out through a feed gas outlet 35 to a respiratory circuit 5 (see FIG. 1). The supply of the liquid in the humidification unit 77 is the same as in the embodiment of the present invention, and so a detailed description will be omitted.

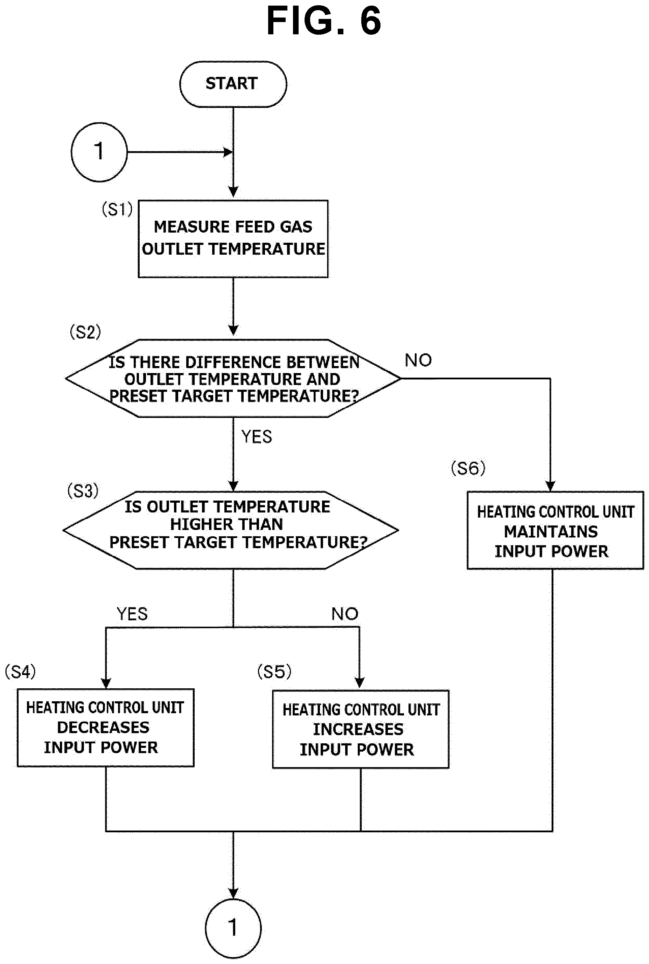

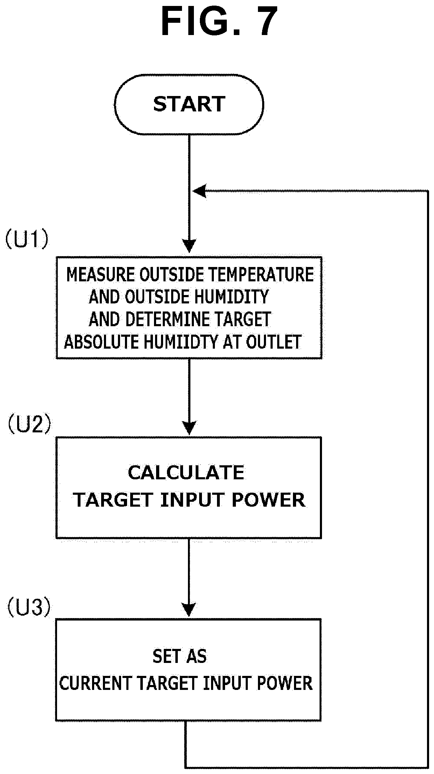

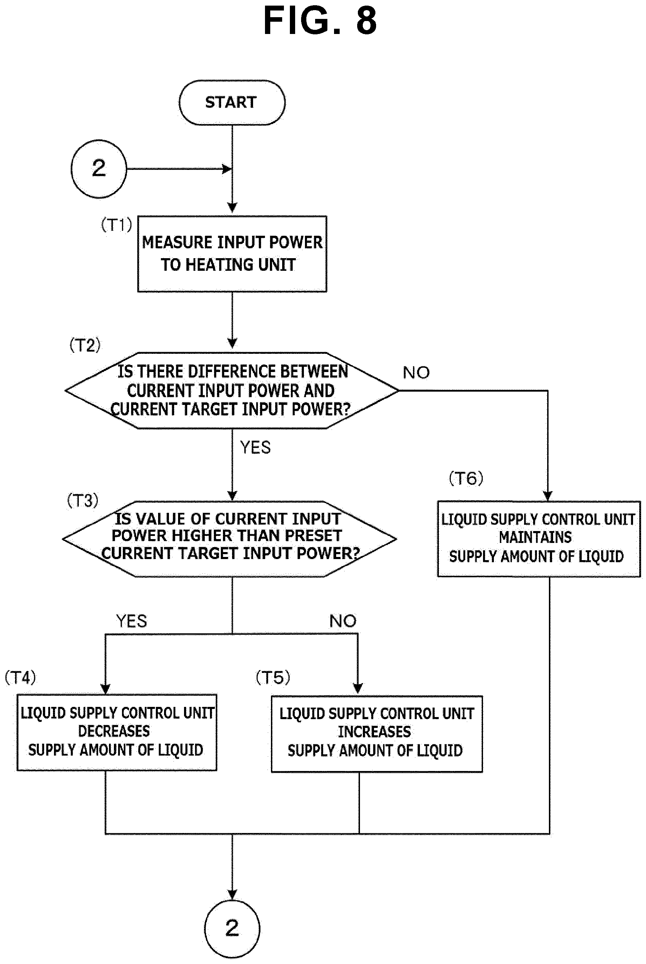

FIG. 5B is a cross-sectional view of the case of the humidification device 9. The warming unit 75 includes a warming coil 79 and a warming heating unit 83. The humidification unit 77 includes a humidification coil 81 and a humidification heating unit 85. The warming unit 75 may be controlled according to an algorithm illustrated in FIG. 6, which will be described later, and the humidification unit 77 may be controlled according to algorithms illustrated in FIGS. 7 and 8, which will be described later.

This variation has the effect of enabling control of temperature and humidity independently.

In this variation, the warming unit 75 is located upstream and the humidification unit 77 is located downstream of the gas supply, but to the contrary, the humidification unit 77 may be located upstream and the warming unit 75 may be located downstream of the gas supply.

In addition, the liquid may be supplied to the warming unit 75 as well as to the humidification unit 77, so that the warming unit 75 may simultaneously heat and humidify the feed gas along with the humidification unit 77.

In general, in the respiratory assistance device 1, it is desirable that the feed gas to be sent into the nasal and oral cavities of the user U have temperatures and relative humidity values determined in advance by a doctor, for example, a temperature of 37°C and a relative humidity of 100%. However, when the feed gas has achieved the above-described determined values at the feed gas outlet 35 of the humidification device 9, the temperature of the feed gas decreases due to heat loss as well as relative humidity, while the feed gas is being sent through the respiratory circuit 5. The degree of heat loss varies depending on an environmental temperature.

Therefore, it is necessary to calculate a target temperature and a target humidity (target absolute humidity) of the feed gas at the feed gas outlet 35 by taking into account environmental variables of an environment in which the respiratory assistance device 1 is placed, i.e., an outside temperature, an outside humidity, and the degree of heat loss in the respiratory circuit 5, and to determine the input power from the power supply 29 and the supply amount of the liquid (water) so as to achieve the target temperature and the target humidity.

Here, in the humidification device 9 in the respiratory assistance device 1 according to the embodiment of the present invention, the target input power calculation unit 17 calculates the target input power by taking into account power required to vaporize water in the heating unit (metal porous body) 59 when the water required to achieve the target absolute humidity is supplied.

Specifically, the humidification device 9 in the respiratory assistance device 1 according to this embodiment is a humidification device configured to humidify a feed gas to be fed to a user U. The humidification device 9 includes the heating unit 59 configured to heat and vaporize a liquid to be used for humidifying the feed gas, the liquid supply unit 63 configured to supply the liquid to the heating unit 59, the power supply 29 configured to supply energy to the heating unit 59, the input power measurement unit 31 configured to measure an input power to be inputted from the power supply 29 to the heating unit 59, the heating control unit 15 configured to control the input power by referring to a temperature in the humidification device 9 or in the respiratory circuit 5 connected to the humidification device 9, the target input power calculation unit 17 configured to calculate a target input power, as a target, corresponding to the target heated and humidified state of the feed gas, and the liquid supply control unit 19 configured to control a supply amount of the liquid on the basis of a difference value between the measured input power and the target input power.

The humidification device 9 in the respiratory assistance device 1 according to this embodiment includes the outside temperature measurement unit 23 configured to measure an outside temperature which is the temperature of an environment in which the user U is present, the outside humidity measurement unit 25 configured to measure an outside humidity which is the humidity of the environment in which the user U is present, and the feed gas outlet temperature measurement unit 7 provided in the vicinity of the feed gas outlet 35 which is an outlet of the feed gas to be sent to the respiratory circuit 5 (see FIG. 1), the geed gas outlet temperature measurement unit being configured to measure a feed gas outlet temperature which is the temperature of the feed gas to be sent to the respiratory circuit 5. Here, the heating control unit 15 controls the input power to the heating unit 59 on the basis of a difference value between the outlet temperature and a preset target temperature, and the target input power calculation unit 17 calculates the target input power on the basis of the values of the outside temperature, the outside humidity, and the outlet temperature.

Furthermore, in the humidification device 9 in the respiratory assistance device 1 according to this embodiment, the liquid supply control unit 19 controls the supply amount of the liquid to the heating unit 59 from the liquid supply amount control unit 65, which varies the supply amount of the liquid.

FIG. 6 is a flowchart that explains an algorithm for controlling the feed gas outlet temperature of the feed gas in the humidification device 9 in the respiratory assistance device 1.

First, a feed gas outlet temperature (hereinafter abbreviated as "outlet temperature") is measured by the feed gas outlet temperature measurement unit 7 (see FIG. 1) disposed at the feed gas outlet 35 of the humidification device 9 (step S1). Whether there is a difference between the outlet temperature and a target temperature that is calculated and set in advance is determined (step S2). In a case where there is no difference, the heating control unit 15 (see FIG. 1) controls to maintain an input power to be measured by the input power measurement unit 31 (see FIG. 1) (step S6). In a case where there is a difference, whether the outlet temperature is higher than the target temperature that is calculated and set in advance is determined (step S3). In a case where the outlet temperature is higher than the target temperature, the heating control unit 15 controls to decrease the input power (Step S4). In a case where the outlet temperature is lower than the target temperature, the heating control unit 15 controls to increase the input power (step S5). After completing the above-described steps S4, S5, and S6, the operation returns to the step S1.

Through the above-described feedback control, the input power is always controlled so that the feed gas outlet temperature remains stable at the target temperature.

The control of the input power by the heating control unit 15 may be PID control that controls, for example, a current value. It is desirable that the specific power control be PWM control.

FIG. 7 is a flowchart that explains an algorithm for calculating and setting a current target input power in real time in the humidification device in the respiratory assistance device.

First, an outside temperature is measured by the outside temperature measurement unit 23 (see FIG. 1), and an outside humidity is measured by the outside humidity measurement unit 25 (see FIG. 1). Then, an absolute humidity, which is a target at the feed gas outlet 35, is determined on the basis of the outside temperature, the outside humidity, a flow rate of the feed gas, a heat loss of the respiratory circuit, and the like (step U1). Next, the amount of water required to achieve this absolute humidity is calculated, and a target input power to be applied from the power supply 29 to the heating unit (metal porous body) 59 to increase the temperature of the amount of water and vaporize the amount of water and, in addition, to increase the temperature of the flowing feed gas is calculated (step U2). The calculated value is then set as a current target input power (step U3). This current target input power is used in an algorithm illustrated in FIG. 8, which will be described later.

By constantly regressing the above-described operation, an optimum target input power can be calculated and set in real time.

FIG. 8 is a flowchart that explains an algorithm for controlling the amount of liquid (water) to be supplied from the power supply 29 to the heating unit 59.

First, an input power to the heating unit (metal porous body) 59 is measured by the input power measurement unit 31 (step T1). Next, whether there is a difference between the current input power and a current target input power is determined (step T2). In a case where there is no difference, the liquid supply control unit 19 controls the liquid supply amount regulation unit 65 (see FIG. 3) to maintain a supply amount of the liquid (step T6). In a case where there is a difference, whether the value of the current input power is larger than the current target input power calculated and set in advance is determined (step T3). In a case where the value of the current input power is larger than the current target input power, the liquid supply control unit 19 controls the liquid supply amount regulation unit 65 to decrease the supply amount of the liquid (step T4). In a case where the value of the current input power is smaller than the current target input power, the liquid supply control unit 19 controls the liquid supply amount regulation unit 65 to increase the supply amount of the liquid (step T5). After completing the above-described steps T4, T5, and T6, the operation returns to step T1.

As a result of the above-described feedback control of the supply amount of the liquid, the input power is controlled to stabilize at the target input power.

In the humidification device 9 in the respiratory assistance device 1 according to this embodiment, the control illustrated in FIG. 6 and the control illustrated in FIG. 8 are performed independently.

In the control of the humidification device 9 in the respiratory assistance device 1 according to the present invention illustrated in FIGS. 6 to 8, the input power of the power supply 29 is determined only by the temperature control based on the feed gas outlet temperature at the feed gas outlet 35. Thus, the input power is controlled to compensate for a drop in the outlet temperature that occurs when the liquid (water) is supplied. On the other hand, the target input power is updated in real time on the basis of the outside environment, the flow rate of the feed gas, the outlet temperature, and the like, and the supply amount of the liquid is controlled so that the actual input power becomes the actual target input power. That is, the feature of this algorithm is that it does not directly control the input power for humidification.

The humidification device 9 in the respiratory assistance device 1 according to the embodiment of the present invention can be configured to spatially separate the humidification unit 71 configured to vaporize the liquid (water) into water vapor, i.e., the heating unit 59, and the energy supply unit 73 configured to supply the energy to the heating unit 59, i.e., the coil 57. Therefore, since only the humidification unit 71, which contains moisture and may cause problems such as easy generation of bacteria, can be replaced with a new one, it is possible to achieve the excellent effect of facilitating maintenance.

According to the humidification device 9 in the respiratory assistance device 1 according to the embodiment of the present invention, since the liquid (water) does not accumulate inside the humidification device 9, it is possible to achieve the excellent effect of making it easier to maintain a good hygiene condition with less chance of bacterial growth.

According to the humidification device 9 in the respiratory assistance device 1 according to the embodiment of the present invention, since the coil 57, which applies energy to the heating unit 59 using the electromagnetic induction phenomenon, and the heating unit 59 are electrically insulated by the insulating unit 61, it is possible to achieve the excellent effect of reducing the possibility of an accident such as a short circuit.

According to the humidification device 9 in the respiratory assistance device 1 according to the embodiment of the present invention, since the coil 57 is disposed inside the cylindrical heating unit 59, it is possible to achieve the excellent effect of efficiently providing the energy from the coil 57 to the heating unit 59 using the electromagnetic induction phenomenon.

According to the humidification device 9 in the respiratory assistance device 1 according to the embodiment of the present invention, since it is easy to increase the surface area in which the heating unit 59 comes into contact with the feed gas to be heated and humidified, it is possible to achieve the excellent effect of providing the humidification device 9 that is compact in size and is capable of sufficient heating and humidification.

The metal porous body has electrical conductivity. According to the humidification device 9 in the respiratory assistance device 1 according to the embodiment of the present invention, it is possible to achieve the excellent effect of generating resistive heat by a flow of electric current through the metal porous body 59 from the coil 57 by the electromagnetic induction phenomenon and thus efficiently vaporizing water.

According to the humidification device 9 in the respiratory assistance device 1 according to the embodiment of the present invention, since the coil and the heating unit 59 through which the electric current flows by electromagnetic induction are efficiently magnetically coupled with each other, it is possible to achieve the excellent effect of increasing energy transfer efficiency from the coil 57 to the heating unit 59.

According to the humidification device 9 in the respiratory assistance device 1 according to the embodiment of the present invention, it is possible to achieve the remarkably excellent effects of enabling the humidification device to be small in size and light in weight, and enabling quick and sufficient humidification without heating an entire body of water to be stored.

According to the humidification device 9 in the respiratory assistance device 1 according to the embodiment of the present invention, it is possible to achieve extremely excellent effects of enabling high-speed heating and humidification control and humidification control with a minimum liquid supply, by independently performing controlling the input power by referring to the temperature in the respiratory circuit 5 connected to the humidification device 9, calculating the target input power, as a target, corresponding to the target heated and humidified state of the feed gas, and controlling the liquid supply amount regulation unit 65 on the basis of the difference value between the input power and the target input power.

According to the humidification device 9 in the respiratory assistance device 1 according to the embodiment of the present invention, since the input power to the heating unit 59 is determined on the basis of environmental variables of the environment in which the user U is present, it is possible to achieve the excellent effect of enabling quick and sufficient heating and humidification with minimum amounts of energy and liquid (water amount).

According to the humidification device 9 in the respiratory assistance device 1 according to the embodiment of the present invention, since the liquid (water) can be supplied to the heating unit 59 only in an amount required for heating and humidification, it is possible to eliminate the accumulation of the liquid water and prevent bacterial growth, which achieves the excellent effect of providing the humidification device which is also excellent in hygienic aspect.

According to the respiratory assistance device 1 according to the embodiment of the present invention, it is possible to achieve the excellent effect of enabling to provide the respiratory assistance device including the compact and lightweight humidification device 9 that can perform quick heating and humidification with the minimum amount of liquid (water amount) and the optimum input power.

Note that, the humidification device and the respiratory assistance device according to the present invention are not limited to the embodiments described above, and as a matter of course, various modifications can be made within the scope of the present invention without departing from the scope of the appended claims.

### Reference Signs List

- 1: respiratory assistance device
- 3: interface
- 5: respiratory circuit
- 7: feed gas outlet temperature measurement unit
- 9: humidification device
- 10: blower
- 13: medical gas cylinder
- 15: heating control unit
- 17: target input power calculation unit
- 19: liquid supply control unit
- 21: control device
- 23: outside temperature measurement unit
- 25: outside humidity measurement unit
- 27: outside environmental variable measurement unit
- 29: power supply
- 30: case
- 31: input power measurement unit
- 35: feed gas outlet
- 40: inlet
- 47: feed gas inlet
- 49: humidification space
- 50: humidifying and heating path
- 51: rectifying plate
- 55: ferrite
- 57: coil
- 59: heating unit (metal porous body)
- 61: insulating unit
- 63: liquid supply unit
- 65: liquid supply amount regulation unit
- 67: liquid storage unit
- 68: liquid supply device
- 69: power supply line
- 71: humidification unit
- 73: energy supply unit
- 75: warming unit
- 77: humidification unit
- 79: warming coil
- 81: humidification coil
- 83: warming heating unit
- 85: humidification heating unit
- 101: respiratory assistance device
- 105: humidification device
- 110: ventilator
- 115: feed gas inlet
- 120: feed gas outlet
- 125: expiratory side respiratory circuit
- 127: inspiratory side respiratory circuit
- 130: hose
- 135: interface
- 140: feed gas outlet temperature measurement unit
- 145: humidification space
- 150: heating element
- 155: liquid (water)
- 160: power supply
- U: user

## Claims

1. A humidification device (9) configured to humidify a feed gas to be fed to a user (U), the humidification device (9) comprising:
a heating unit (59) configured to heat and vaporize a liquid to be used for humidifying the feed gas;
a liquid supply unit (63) configured to supply the liquid to the heating unit (59);
a power supply (29) configured to supply energy to the heating unit (59); and
an input power measurement unit (31) configured to measure an input power to be inputted from the power supply (29) to the heating unit (59),
**characterized by**
a heating control unit (15) configured to control the input power by referring to a temperature of the feed gas in the humidification device (9) or in a respiratory circuit (5) connected to the humidification device (9);
a target input power calculation unit (17) configured to calculate a target input power, as a target, corresponding to a target heated and humidified state of the feed gas; and
a liquid supply control unit (19) configured to decrease a supply amount of the liquid when the measured input power is greater than the target input power and increase the supply amount of the liquid when the measured input power is less than the target input power.

2. The humidification device (9) according to claim 1, **characterized by** comprising:
an outside temperature measurement unit (23) configured to measure an outside temperature which is a temperature of an environment in which the user (U) is present; and
an outside humidity measurement unit (25) configured to measure an outside humidity which is a humidity of the environment in which the user (U) is present,
wherein the target input power calculation unit (17) calculates the target input power on a basis of at least values of the outside temperature, and the outside humidity.

3. The humidification device (9) according to claim 1 or 2, **characterized by** comprising:
a case (30) which contains a humidification space (49) in which water vapor is introduced to a feed gas to be fed to a user (U);
a coil (57) configured to transfer energy to the heating unit (59) by the electromagnetic induction phenomenon;
an insulating unit (61) configured to spatially separate the heating unit (59) and the coil (57) to prevent electrical contact therebetween.

4. The humidification device (9) according to any one of claims 1 to 3, **characterized in that** the liquid supply unit (63) supplies the liquid so as to maintain a state in which the liquid does not accumulate in a vicinity of the heating unit (59).

5. The humidification device (9) according to claim 3, **characterized in that** the heating unit (59) has a cylindrical shape, and the coil (57) is disposed via the insulating unit (61).

6. The humidification device (9) according to claim 5, **characterized in that** the insulating unit (61) has a cylindrical shape and is disposed on an inner peripheral side of the heating unit (59), and the coil (57) is disposed on an inner peripheral side of the insulating unit (61).

7. The humidification device (9) according to claim 1 or 6, **characterized by** further comprising a liquid supply amount regulation unit (65) configured to regulate a supply amount of the liquid by the liquid supply unit (63), wherein the liquid supply control unit (19) controls the liquid supply amount regulation unit.

8. The humidification device (9) according to claim 7, **characterized in that** the supply amount of liquid regulated by the liquid supply amount regulation unit (65), is set within a range not exceeding the maximum amount of liquid that the heating unit (59) can vaporize.

9. A respiratory assistance device (1) **characterized by** comprising the humidification device (9) according to any one of claims 1 to 8.

10. A humidification method by a humidification device (9) for humidifying a feed gas, the humidification device (9) including:
a heating unit (59) configured to heat and vaporize a liquid to be used for humidifying the feed gas to be fed to a user (U);
a liquid supply unit (63) configured to supply the liquid to the heating unit (59);
a power supply (29) configured to supply energy to the heating unit (59); and
an input power measurement unit (31) configured to measure an input power to be inputted from the power supply (29) to the heating unit (59),
the humidification method **characterized by:**
a heating controlling step of controlling the input power by referring to a temperature of the feed gas in the humidification device (9) or in a respiratory circuit (5) connected to the humidification device (9);
a target input power calculating step of calculating a target input power, as a target, corresponding to a target heated and humidified state of the feed gas; and
a liquid supply controlling step of decreasing a supply amount of the liquid when the measured input power is greater than the target input power and increasing the supply amount of the liquid when the measured input power is less than the target input power.

11. The humidification method according to claim 10, **characterized in that:**
the humidification device (9) includes
an outside temperature measurement unit (23) configured to measure an outside temperature which is a temperature of an environment in which the user (U) is present, and
an outside humidity measurement unit (25) configured to measure an outside humidity which is a humidity of the environment in which the user (U) is present,
wherein, in the target input power calculation step, the target input power is calculated on a basis of at least values of the outside temperature, and the outside humidity.

12. The humidification method according to claim 10 or 11, **characterized in that** the humidification device (9) further comprising a liquid supply amount regulation unit configured to regulate a supply amount of the liquid by the liquid supply unit (63), wherein in the liquid supply controlling step, the liquid supply amount regulation unit is controlled.

## Patentansprüche

1. Befeuchtungsvorrichtung (9), die konfiguriert ist, um ein zu einem Benutzer (U) zuzuführendes Zufuhrgas zu befeuchten, wobei die Befeuchtungsvorrichtung (9) aufweist:
eine Heizeinheit (59), die konfiguriert ist, um eine zum Befeuchten des Zufuhrgases zu verwendende Flüssigkeit zu erhitzen und zu verdampfen;
eine Flüssigkeitszufuhreinheit (63), die konfiguriert ist, um die Flüssigkeit der Heizeinheit (59) zuzuführen;
eine Energieversorgung (29), die konfiguriert ist, um der Heizeinheit (59) Energie zuzuführen; und
eine Eingangsleistungsmesseinheit (31), die konfiguriert ist, um eine Eingangsleistung zu messen, die von der Energieversorgung (29) in die Heizeinheit (59) einzuspeisen ist,
**gekennzeichnet durch**
eine Heizsteuereinheit (15), die konfiguriert ist, um die Eingangsleistung unter Bezugnahme auf eine Temperatur des Zufuhrgases in der Befeuchtungsvorrichtung (9) oder in einem mit der Befeuchtungsvorrichtung (9) verbundenen Beatmungskreislauf (5) zu steuern;
eine Solleingangsleistungsberechnungseinheit (17), die konfiguriert ist, um eine Solleingangsleistung als ein Ziel zu berechnen, das einem Sollerhitzungs- und Befeuchtungszustand des Zufuhrgases entspricht; und
eine Flüssigkeitszufuhrsteuereinheit (19), die konfiguriert ist, um eine Zufuhrmenge der Flüssigkeit zu verringern, wenn die gemessene Eingangsleistung größer als die Solleingangsleistung ist, und die Zufuhrmenge der Flüssigkeit zu erhöhen, wenn die gemessene Eingangsleistung kleiner als die Solleingangsleistung ist.

2. Befeuchtungsvorrichtung (9) gemäß Anspruch 1, **gekennzeichnet durch:**
eine Außentemperaturmesseinheit (23), die konfiguriert ist, um eine Außentemperatur zu messen, die eine Temperatur einer Umgebung ist, in der sich der Benutzer (U) befindet; und
eine Außenfeuchtigkeitsmesseinheit (25), die konfiguriert ist, um eine Außenfeuchtigkeit zu messen, die eine Feuchtigkeit der Umgebung ist, in der sich der Benutzer (U) befindet,
wobei die Solleingangsleistungsberechnungseinheit (17) die Solleingangsleistung auf der Grundlage von mindestens Werten der Außentemperatur und der Außenfeuchtigkeit berechnet.

3. Befeuchtungsvorrichtung (9) gemäß Anspruch 1 oder 2, **gekennzeichnet durch:**
ein Gehäuse (30), das einen Befeuchtungsraum (49) enthält, in dem Wasserdampf in ein Zufuhrgas eingebracht wird, das einem Benutzer (U) zuzuführen ist;
eine Spule (57), die konfiguriert ist, um durch das Phänomen der elektromagnetischen Induktion Energie auf die Heizeinheit (59) zu übertragen;
eine Isolierungseinheit (61), die konfiguriert ist, um die Heizeinheit (59) und die Spule (57) räumlich voneinander zu trennen, um einen elektrischen Kontakt zwischen diesen zu verhindern.

4. Befeuchtungsvorrichtung (9) gemäß einem der Ansprüche 1 bis 3, **dadurch gekennzeichnet, dass** die Flüssigkeitszufuhreinheit (63) die Flüssigkeit so zuführt, dass ein Zustand aufrechterhalten wird, in dem sich die Flüssigkeit nicht in der Nähe der Heizeinheit (59) ansammelt.

5. Befeuchtungsvorrichtung (9) gemäß Anspruch 3, **dadurch gekennzeichnet, dass** die Heizeinheit (59) eine zylindrische Form aufweist und die Spule (57) über die Isolierungseinheit (61) angeordnet ist.

6. Befeuchtungsvorrichtung (9) gemäß Anspruch 5, **dadurch gekennzeichnet, dass** die Isolierungseinheit (61) eine zylindrische Form aufweist und an einer inneren Umfangsseite der Heizeinheit (59) angeordnet ist und die Spule (57) an einer inneren Umfangsseite der Isolierungseinheit (61) angeordnet ist.

7. Befeuchtungsvorrichtung (9) gemäß Anspruch 1 oder 6, **gekennzeichnet durch** eine Flüssigkeitszufuhrmengenreguliereinheit (65), die konfiguriert ist, um eine Zufuhrmenge der Flüssigkeit durch die Flüssigkeitszufuhreinheit (63) zu regulieren, wobei die Flüssigkeitszufuhrsteuereinheit (19) die Flüssigkeitszufuhrmengenreguliereinheit steuert.

8. Befeuchtungsvorrichtung (9) gemäß Anspruch 7, **dadurch gekennzeichnet, dass** die von der Flüssigkeitszufuhrmengenreguliereinheit (65) regulierte Flüssigkeitszufuhrmenge innerhalb eines Bereichs eingestellt ist, der die maximale Flüssigkeitsmenge, die die Heizeinheit (59) verdampfen kann, nicht überschreitet.

9. Atmungsunterstützungsvorrichtung (1), **gekennzeichnet durch** die Befeuchtungsvorrichtung (9) gemäß einem der Ansprüche 1 bis 8.

10. Befeuchtungsverfahren durch eine Befeuchtungsvorrichtung (9) zum Befeuchten eines Zufuhrgases, wobei die Befeuchtungsvorrichtung (9) umfasst:
eine Heizeinheit (59), die konfiguriert ist, um eine zum Befeuchten des Zufuhrgases zu verwendende Flüssigkeit zu erhitzen und zu verdampfen;
eine Flüssigkeitszufuhreinheit (63), die konfiguriert ist, um die Flüssigkeit der Heizeinheit (59) zuzuführen;
eine Energieversorgung (29), die konfiguriert ist, um der Heizeinheit (59) Energie zuzuführen; und
eine Eingangsleistungsmesseinheit (31), die konfiguriert ist, um eine Eingangsleistung zu messen, die von der Energieversorgung (29) in die Heizeinheit (59) einzuspeisen ist,
wobei, das Befeuchtungsverfahren ist **gekennzeichnet durch:**
einen Heizsteuerschritt zum Steuern der Eingangsleistung durch Bezugnahme auf eine Temperatur des Zufuhrgases in der Befeuchtungsvorrichtung (9) oder in einem mit der Befeuchtungsvorrichtung (9) verbundenen Beatmungskreislauf (5);
einen Solleingangsleistungsberechnungsschritt zum Berechnen einer Solleingangsleistung als Ziel, das einem Sollerwärmungs- und - Befeuchtungszustand des Zufuhrgases entspricht; und
einen Schritt zur Steuerung der Flüssigkeitszufuhr, bei dem die Zufuhrmenge der Flüssigkeit verringert wird, wenn die gemessene Eingangsleistung größer ist als die Solleingangsleistung, und die Zufuhrmenge der Flüssigkeit erhöht wird, wenn die gemessene Eingangsleistung geringer ist als die Solleingangsleistung.

11. Befeuchtungsverfahren gemäß Anspruch 10, **dadurch gekennzeichnet, dass** die Befeuchtungsvorrichtung (9) umfasst
eine Außentemperaturmesseinheit (23), die konfiguriert ist, um eine Außentemperatur zu messen, die eine Temperatur einer Umgebung ist, in der sich der Benutzer (U) befindet; und
eine Außenfeuchtigkeitsmesseinheit (25), die konfiguriert ist, um eine Außenfeuchtigkeit zu messen, die eine Feuchtigkeit der Umgebung ist, in der sich der Benutzer (U) befindet,
wobei in dem Solleingangsleistungsberechnungsschritt die Solleingangsleistung auf der Grundlage von mindestens Werten der Außentemperatur und der Außenfeuchtigkeit berechnet wird.

12. Befeuchtungsverfahren gemäß Anspruch 10 oder 11, **dadurch gekennzeichnet, dass** die Befeuchtungsvorrichtung (9) weiterhin eine Flüssigkeitszufuhrmengenreguliereinheit umfasst, die konfiguriert ist, um eine Zufuhrmenge der Flüssigkeit durch die Flüssigkeitszufuhreinheit (63) zu regulieren, wobei in dem Flüssigkeitszufuhrsteuerungsschritt die Flüssigkeitszufuhrmengenreguliereinheit gesteuert wird.

## Revendications

1. Dispositif d'humidification (9) configuré pour humidifier un gaz d'alimentation destiné à être administré à un utilisateur (U), le dispositif d'humidification (9) comprenant :
une unité de chauffage (59) configurée pour chauffer et vaporiser un liquide à utiliser pour humidifier le gaz d'alimentation ;
une unité d'alimentation en liquide (63) configurée pour fournir le liquide à l'unité de chauffage (59) ; et
une alimentation en énergie (29) configurée pour fournir de l'énergie à l'unité de chauffage (59) ; et
une unité de mesure de la puissance d'entrée (31) configurée pour mesurer une puissance d'entrée à introduire par l'alimentation en énergie (29) à l'unité de chauffage (59),
**caractérisé par**
une unité de contrôle de chauffage (15) configurée pour contrôler la puissance d'entrée en se référant à une température du gaz d'alimentation dans le dispositif d'humidification (9) ou dans un circuit respiratoire (5) relié au dispositif d'humidification (9) ;
une unité de calcul de la puissance d'entrée cible (17) configurée pour calculer une puissance d'entrée cible, en tant que cible, correspondant à un état chauffé et humidifié cible du gaz d'alimentation ; et
une unité de contrôle de l'alimentation en liquide (19) configurée pour diminuer une quantité d'alimentation en liquide lorsque la puissance d'entrée mesurée est supérieure à la puissance d'entrée cible et pour augmenter la quantité d'alimentation en liquide lorsque la puissance d'entrée mesurée est inférieure à la puissance d'entrée cible.

2. Dispositif d'humidification (9) selon la revendication 1, **caractérisé en ce qu'**il comprend :
une unité de mesure de la température extérieure (23) configurée pour mesurer une température extérieure qui est une température d'un environnement dans lequel l'utilisateur (U) est présent ; et
une unité de mesure de l'humidité extérieure (25) configurée pour mesurer une humidité extérieure qui est une humidité de l'environnement dans lequel l'utilisateur (U) est présent,
dans lequel l'unité de calcul de la puissance d'entrée cible (17) calcule la puissance d'entrée cible sur la base d'au moins les valeurs de la température extérieure, et de l'humidité extérieure.

3. Dispositif d'humidification (9) selon la revendication 1 ou 2, **caractérisé en ce qu'**il comprend :
un boîtier (30) qui contient un espace d'humidification (49) dans lequel de la vapeur d'eau est introduite dans un gaz d'alimentation destiné à être administré à un utilisateur (U) ;
une bobine (57) configurée pour transférer de l'énergie à l'unité de chauffage (59) par le phénomène d'induction électromagnétique ;
une unité isolante (61) configurée pour séparer dans l'espace l'unité chauffante (59) et la bobine (57) afin d'éviter tout contact électrique entre elles.

4. Dispositif d'humidification (9) selon l'une quelconque des revendications 1 à 3, **caractérisé en ce que** l'unité d'alimentation en liquide (63) fournit le liquide de manière à maintenir un état dans lequel le liquide ne s'accumule pas à proximité de l'unité de chauffage (59).

5. Dispositif d'humidification (9) selon la revendication 3, **caractérisé en ce que** l'unité de chauffage (59) a une forme cylindrique, et la bobine (57) est disposé via l'unité isolante (61).

6. Dispositif d'humidification (9) selon la revendication 5, **caractérisé en ce que** l'unité isolante (61) a une forme cylindrique et est disposée sur un côté périphérique intérieur de l'unité de chauffage (59), et la bobine (57) est disposée sur un côté périphérique intérieur de l'unité isolante (61).

7. Dispositif d'humidification (9) selon la revendication 1 ou 6, **caractérisé en ce qu'**il comprend en outre une unité de régulation de la quantité d'alimentation en liquide (65) configurée pour réguler une quantité d'alimentation en liquide par l'unité d'alimentation en liquide (63), l'unité de contrôle d'alimentation en liquide (19) contrôlant l'unité de régulation de la quantité d'alimentation en liquide.

8. Dispositif d'humidification (9) selon la revendication 7, **caractérisé en ce que** la quantité d'alimentation en liquide régulée par l'unité de régulation de la quantité d'alimentation en liquide (65), est réglée dans une plage ne dépassant pas la quantité maximale de liquide que l'unité de chauffage (59) peut vaporiser.

9. Appareil d'assistance respiratoire (1) **caractérisé en ce qu'**il comprend le dispositif d'humidification (9) selon l'une quelconque des revendications 1 à 8.

10. Procédé d'humidification par un dispositif d'humidification (9) pour humidifier un gaz d'alimentation, le dispositif d'humidification (9) comportant :
une unité de chauffage (59) configurée pour chauffer et vaporiser un liquide à utiliser pour humidifier le gaz d'alimentation destiné à être administré à un utilisateur (U) ;
une unité d'alimentation en liquide (63) configurée pour fournir le liquide à l'unité de chauffage (59) ;
une alimentation en énergie (29) configurée pour fournir de l'énergie à l'unité de chauffage (59) ; et
une unité de mesure de la puissance d'entrée (31) configurée pour mesurer une puissance d'entrée à introduire par l'alimentation en énergie (29) à l'unité de chauffage (59),
le procédé d'humidification **caractérisé par**:
une étape de contrôle du chauffage consistant à contrôler la puissance d'entrée en se référant à une température du gaz d'alimentation dans le dispositif d'humidification (9) ou dans un circuit respiratoire (5) connecté au dispositif d'humidification (9) ;
une étape de calcul de la puissance d'entrée cible consistant à calculer une puissance d'entrée cible, en tant que cible, correspondant à un état chauffé et humidifié cible du gaz d'alimentation ; et
une étape de contrôle de l'alimentation en liquide consistant à diminuer une quantité d'alimentation en liquide lorsque la puissance d'entrée mesurée est supérieure à la puissance d'entrée cible et à augmenter la quantité d'alimentation en liquide lorsque la puissance d'entrée mesurée est inférieure à la puissance d'entrée cible.

11. Procédé d'humidification selon la revendication 10, **caractérisée en ce que**:
le dispositif d'humidification (9) comporte
une unité de mesure de la température extérieure (23) configurée pour mesurer une température extérieure qui est une température d'un environnement dans lequel l'utilisateur (U) est présent, et
une unité de mesure de l'humidité extérieure (25) configurée pour mesurer une humidité extérieure qui est une humidité de l'environnement dans lequel l'utilisateur (U) est présent,
dans lequel, dans l'étape de calcul de la puissance d'entrée cible, la puissance d'entrée cible est calculée sur la base d'au moins les valeurs de la température extérieure, et de l'humidité extérieure.

12. Procédé d'humidification selon la revendication 10 ou 11, **caractérisé en ce que** le dispositif d'humidification (9) comprend en outre une unité de régulation de la quantité d'alimentation en liquide configurée pour réguler une quantité d'alimentation en liquide par l'unité d'alimentation en liquide (63), dans lequel, dans l'étape de contrôle de l'alimentation en liquide, l'unité de régulation de la quantité d'alimentation en liquide est contrôlée.
